(19) Europäisches Patentamt
European Patent Office
Office européen des brevets

(11) Veröffentlichungsnummer: **0 351 676 A2**

(12) **EUROPÄISCHE PATENTANMELDUNG**

(21) Anmeldenummer: **89112508.0**

(51) Int. Cl.4: **C07D 417/04 , A01N 43/80**

(22) Anmeldetag: **08.07.89**

(30) Priorität: **21.07.88 JP 180419/88**
**22.10.88 JP 266663/88**

(43) Veröffentlichungstag der Anmeldung:
**24.01.90 Patentblatt 90/04**

(84) Benannte Vertragsstaaten:
**BE CH DE FR GB IT LI NL**

(71) Anmelder: **NIHON TOKUSHU NOYAKU SEIZO K.K.**
**Itohpla Nihonbashi Honcho Building 7-1,**
**Nihonbashi Honcho 2-chome**
**Chuo-ku Tokyo 103(JP)**

(72) Erfinder: **Kume, Toyohiko**
**6-7-8, Asahigaoka**
**Hino-shi Tokyo(JP)**
Erfinder: **Goto, Toshio**
**3454-21, Honmachida**
**Machida-shi Tokyo(JP)**
Erfinder: **Kamochi, Atsumi**
**2-24-10, Higashi-Toyoda**
**Hino-shi Tokyo(JP)**
Erfinder: **Yanagi, Akihiko**
**2-1200-1, Nagabuchi**
**Oume-shi Tokyo(JP)**
Erfinder: **Yagi, Shigeki**
**1-30-7, Izumi Suginami-ku**
**Tokyo(JP)**
Erfinder: **Miyauchi, Hiroshi**
**39-15, Namiki-cho**
**Hachioji-shi Tokyo(JP)**

(74) Vertreter: **Schumacher, Günter, Dr. et al**
**c/o Bayer AG Konzernverwaltung RP**
**Patentabteilung**
**D-5090 Leverkusen 1 Bayerwerk(DE)**

(54) **Benzothiazolone.**

(57) Offenbart werden neue Benzothiazolone der Formel (I)

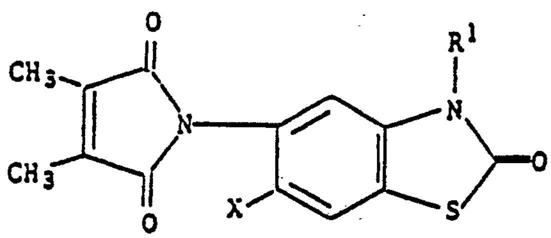

(I)

Verfahren zur Herstellung dieser neuen Verbindungen sowie deren Verwendung als Herbizide.

EP 0 351 676 A2

Xerox Copy Centre

## Benzothiazolone

Die vorliegende Erfindung betrifft neue Benzothiazolone, Verfahren zu ihrer Herstellung und ihre Verwendung als Herbizide.

Es wurde bereits offenbart, daß bestimmte 3,4-Dimethylmaleinimide als Zwischenprodukte für herbizid wirksame 3,4-Dimethyl-2-hydroxy-5-oxo-2,5-dihydropyrrole verwendet wurden (siehe die US-PS 4 138 243) und daß auch bestimmte Benzothiazolone eine herbizide Funktion aufweisen (siehe die JP-OS 155 276/1987).

Nunmehr wurden neue Benzothiazolone der nachstehenden Formel (I)

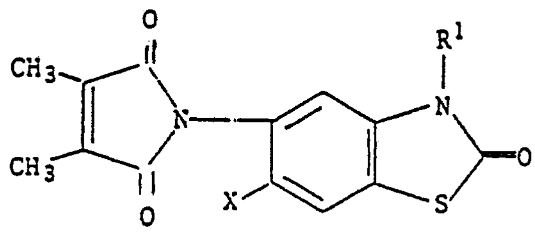

(I)

gefunden, in der

X Wasserstoff oder Halogen darstellt,

$R^1$ Wasserstoff, Alkyl mit 1 bis 5 Kohlenstoff-Atomen, das gegebenenfalls mit Halogen substituiert ist, Alkenyl mit 3 bis 5 Kohlenstoff-Atomen, das gegebenenfalls mit Halogen substituiert ist, Alkinyl mit 3 bis 4 Kohlenstoff-Atomen, das gegebenenfalls mit Halogen substituiert ist, Alkoxyalkyl mit insgesamt 2 bis 5 Kohlenstoff-Atomen, Alkylthioalkyl mit insgesamt 2 bis 5 Kohlenstoff-Atomen, Alkylsulfinylalkyl mit insgesamt 2 bis 5 Kohlenstoff-Atomen, Alkylsulfonylalkyl mit insgesamt 2 bis 5 Kohlenstoff-Atomen, Arylthioalkyl mit insgesamt 7 bis 8 Kohlenstoff-Atomen, vorzugsweise Phenylthioalkyl mit 1 oder 2 Kohlenstoff-Atomen im Alkyl-Teil und gegebenenfalls mit Halogen substituiert, Cyanoalkyl mit einer Alkyl-Struktureinheit mit 1 bis 2 Kohlenstoff-Atomen, Carbamoylmethyl, Thiocarbamoylmethyl, Alkoxycarbonylalkyl mit insgesamt 3 bis 8 Kohlenstoff-Atomen, Cycloalkoxycarbonylmethyl mit einer Cycloalkyl-Struktureinheit mit 3 bis 7 Kohlenstoff-Atomen, Trialkylsilylmethyl mit insgesamt 4 bis 10 Kohlenstoff-Atomen oder einen Rest einer der folgenden Formeln

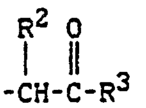 , 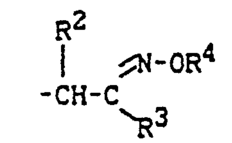 und 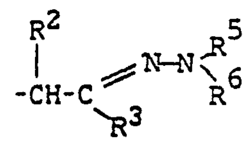

darstellt, in denen

$R^2$ Wasserstoff oder Alkyl mit 1 bis 3 Kohlenstoff-Atomen darstellt,

$R^3$ Alkyl mit 1 bis 4 Kohlenstoff-Atomen, Cycloalkyl mit 3 bis 6 Kohlenstoff-Atomen oder Phenyl, das gegebenenfalls mit Halogen substituiert ist, darstellt,

$R^4$ Wasserstoff, Alkyl mit 1 bis 4 Kohlenstoff-Atomen, Alkenyl mit 3 bis 4 Kohlenstoff-Atomen, Alkinyl mit 3 bis 4 Kohlenstoff-Atomen, Aralkyl mit insgesamt 7 bis 9 Kohlenstoff-Atomen, vorzugsweise Phenylalkyl mit 1 bis 3 Kohlenstoff-Atomen im Alkyl-Teil, Alkylcarbonyl mit einer Alkyl-Struktureinheit mit 1 bis 4 Kohlenstoff-Atomen oder Alkansulfonyl mit 1 bis 4 Kohlenstoff-Atomen darstellt und

$R^5$ und $R^6$ jeweils Wasserstoff oder Alkyl mit 1 bis 4 Kohlenstoff-Atomen darstellen.

Benzothiazolone der Formel (I) werden erhalten, wenn

a) Verbindungen Formel (Ia)

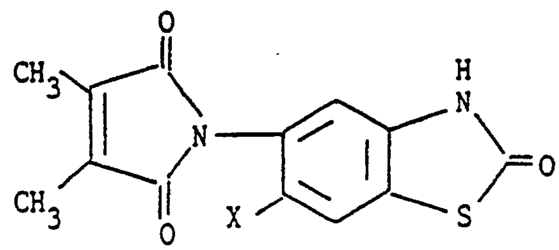

(Ia)

,

in der
X die im Vorstehenden angegebenen Bedeutungen hat,
mit Verbindungen der Formel (III)

$M^1 - R^1$ (III),

in der
$R^1$ die im Vorstehenden angegebenen Bedeutungen hat und
$M^1$ Halogen ist,
in Gegenwart inerter Lösungsmittel und erforderlichenfalls in Gegenwart von Basen umgesetzt werden, oder

b) Verbindungen der Formel (IV)

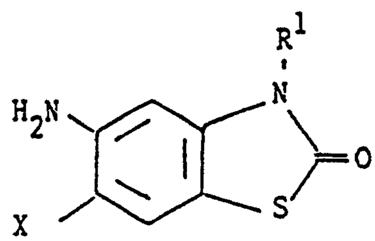

(IV)

,

in der
X und $R^1$ die im Vorstehenden angegebenen Bedeutungen haben,
mit 2,3-Dimethylmaleinsäureanhydrid in Gegenwart inerter Lösungsmittel umgesetzt werden, oder

c) in dem Fall, in dem $R^1$ Alkylsulfinlyalkyl mit insgesamt 2 bis 5 Kohlenstoff-Atomen oder Alkylsulfonlyalkyl mit insgesamt 2 bis 5 Kohlenstoff-Atomen ist, Verbindungen der Formel (Ib)

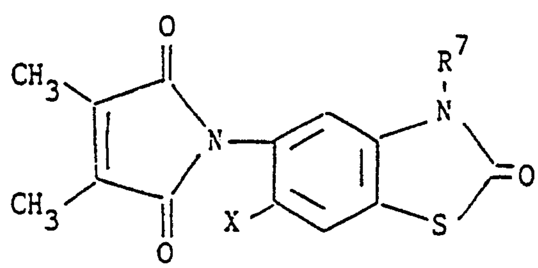

(Ib)

,

in der
X die im Vorstehenden angegebenen Bedeutungen hat und
$R^7$ Alkylthioalkyl mit insgesamt 2 bis 5 Kohlenstoff-Atomen ist,
in Gegenwart inerter Lösungsmittel oxidiert werden, oder

d) in dem Fall, in dem $R^1$

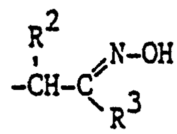

ist,
Verbindungen der Formel (Ic)

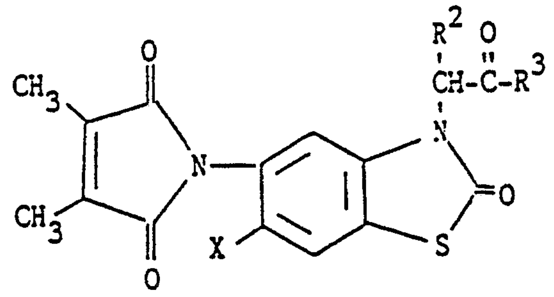

(Ic)

,

in der
X, $R^2$ und $R^3$ die im Vorstehenden angegebenen Bedeutungen haben,
mit Hydroxylamin in Gegenwart inerter Lösungsmittel umgesetzt werden, oder

e) in dem Fall, in dem $R^1$

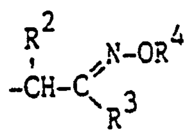

ist,
Verbindungen der Formel (Id)

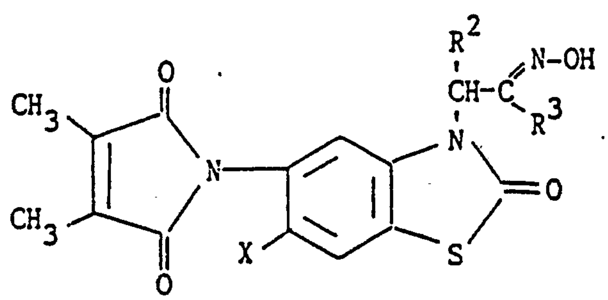

(Id)

,

in der
X, $R^2$ und $R^3$ die im Vorstehenden angegebenen Bedeutungen haben,
mit Verbindungen der Formel (VII)
$M^1$ - $R^4$ (VII),
in der
$R^4$ und $M^1$ die im Vorstehenden angegebenen Bedeutungen haben,
in Gegenwart inerter Lösungsmittel und erforderlichenfalls in Gegenwart von Basen umgesetzt werden.

Die neuen Benzothiazolone zeigen potente herbizide Eigenschaften.

Überraschenderweise zeigen die erfindungsgemäßen Benzothiazolone eine wesentlich stärkere herbizide Wirkung als die aus dem oben genannten Stand der Technik bekannten Verbindungen, wie im einzelnen aus den folgenden Beispielen hervorgeht.

Die Angabe "gegebenenfalls mit Halogen substituiert" bedeutet, daß der betreffende Rest einfach oder mehrfach mit gleichen oder verschiedenen Halogen-Atomen aus Fluor, Chlor, Brom oder Iod, vorzugsweise mit Fluor oder Chlor und insbesondere durch Chlor, substituiert sein kann. Eine wahlweise Substitution durch Chlor bedeutet, daß der Rest einfach oder mehrfach mit Chlor, vorzugsweise bis fünffach und insbsesondere bis dreifach mit Chlor substituiert sein kann

Unter den erfindungsgemäßen Benzothiazolonen der Formel (I) sind bevorzugte Verbindungen diejenigen, in denen X Wasserstoff oder Fluor darstellt,

$R^1$ Wasserstoff, Alkyl mit 1 bis 4 Kohlenstoff-Atomen, das gegebenenfalls mit Chlor substituiert ist, Alkenyl mit 3 bis 4 Kohlenstoff-Atomen, das gegebenenfalls mit Chlor substituiert ist, Alkinyl mit 3 bis 4 Kohlenstoff-Atomen, das gegebenenfalls mit Chlor substituiert ist, Alkoxyalkyl mit insgesamt 2 bis 4 Kohlenstoff-Atomen, Alkylthioalkyl mit insgesamt 2 bis 3 Kohlenstoff-Atomen, Alkylsulfinylalkyl mit insgesamt 2 bis 4 Kohlenstoff-Atomen, Alkylsulfonylalkyl mit insgesamt 2 bis 4 Kohlenstoff-Atomen, Phenylthiomethyl, das gegebenenfalls im Phenyl-Teil mit Chlor substituiert ist, Cyanomethyl, Carbamoylmethyl, Thiocarbamoylmethyl, Alkoxycarbonylalkyl mit insgesamt 3 bis 6 Kohlenstoff-Atomen, Cycloalkoxycarbonylmethyl mit einer Cycloalkyl-Struktureinheit mit 3 bis 6 Kohlenstoff-Atomen, Trimethylsilylmethyl oder einen Rest einer der folgenden Formeln

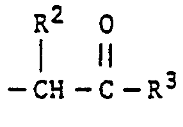 , 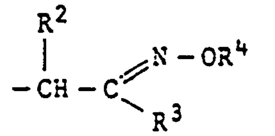 und 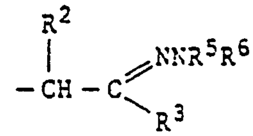

darstellt, in denen

$R^2$ Wasserstoff, Methyl oder Ethyl darstellt,

$R^3$ Alkyl mit 1 bis 3 Kohlenstoff-Atomen, Cycloalkyl mit 3 bis 5 Kohlenstoff-Atomen oder Phenyl, das gegebenenfalls mit Fluor und/oder mit Chlor substituiert ist, darstellt,

$R^4$ Wasserstoff, Alkyl mit 1 bis 3 Kohlenstoff-Atomen, Allyl, Propargyl, Benzyl, Alkylcarbonyl mit einer Alkyl-Struktureinheit mit 1 bis 3 Kohlenstoff-Atomen oder Alkansulfonyl mit 1 bis 2 Kohlenstoff-Atomen darstellt und

$R^5$ und $R^6$ jeweils Wasserstoff oder Alkyl mit 1 bis 2 Kohlenstoff-Atomen darstellen.

Ganz besonders bevorzugte Benzothiazolone der Formel (I) sind diejenigen, in denen

X Fluor darstellt,

$R^1$ Wasserstoff, Alkyl mit 1 bis 3 Kohlenstoff-Atomen, das gegebenenfalls mit Chlor substituiert ist, Allyl, das gegebenenfalls mit Chlor substituiert ist, Propargyl, Alkoxymethyl mit einer Alkoxy-Struktureinheit mit 1 bis 3 Kohlenstoff-Atomen, Alkylthiomethyl mit einer Alkylmercapto-Struktureinheit mit 1 bis 2 Kohlenstoff-Atomen, Alkylsulfinylmethyl mit einer Alkylsulfinyl-Struktureinheit mit 1 bis 2 Kohlenstoff-Atomen, Alkylsulfonylmethyl mit einer Alkylsulfonyl-Struktureinheit mit 1 bis 2 Kohlenstoff-Atomen, Cyanomethyl oder einen Rest der Formel

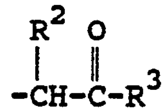

darstellt, in denen

$R^2$ Wasserstoff oder Methyl darstellt und

$R^3$ Methyl oder Ethyl darstellt.

Speziell genannt seien die folgenden Verbindungen:

5-(3,4-Dimethylmaleinimido)-6-fluoro-3-propargyl-2-benzothiazolon,
3-Allyl-5-(3,4-dimethylmaleinimido)-6-fluoro-2-benzothiazolon,
5-(3,4-Dimethylmaleinimido)-6-fluoro-3-propyl-2-benzothiazolon,
3-Cyanomethyl-5-(3,4-dimethylmaleinimido)-6-fluoro-2-benzothiazolon,
5-(3,4-Dimethylmaleinimido)-6-fluoro-3-methylthiomethyl-2-benzothiazolon,
5-(3,4-Dimethylmaleinimido)-6-fluoro-3-methylsulfinylmethyl-2-benzothiazolon und
5-(3,4-Dimethylmaleinimido)-6-fluoro-3-methylsulfonylmethyl-2-benzothiazolon.

Wenn die in dem Verfahren (a) eingesetzten Ausgangsstoffe beispielsweise 5-(3,4-Dimethylmaleinimido)-6-fluoro-2-benzothiazolon und Propargylbromid sind, kann die Reaktion durch das folgende Schema dargestellt werden:

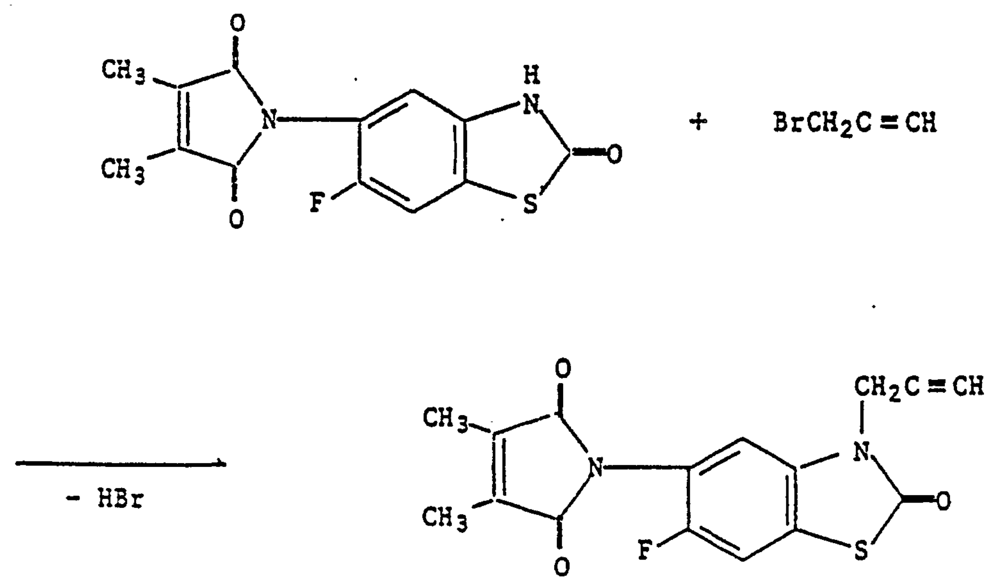


Wenn die in dem Verfahren (b) eingesetzten Ausgangsstoffe beispielsweise 5-Amino-6-fluoro-3-propargyl-2-benzothiazolon und 2,3-Dimethylmaleinsäureanhydrid sind, kann die Reaktion durch das folgende Schema dargestellt werden:

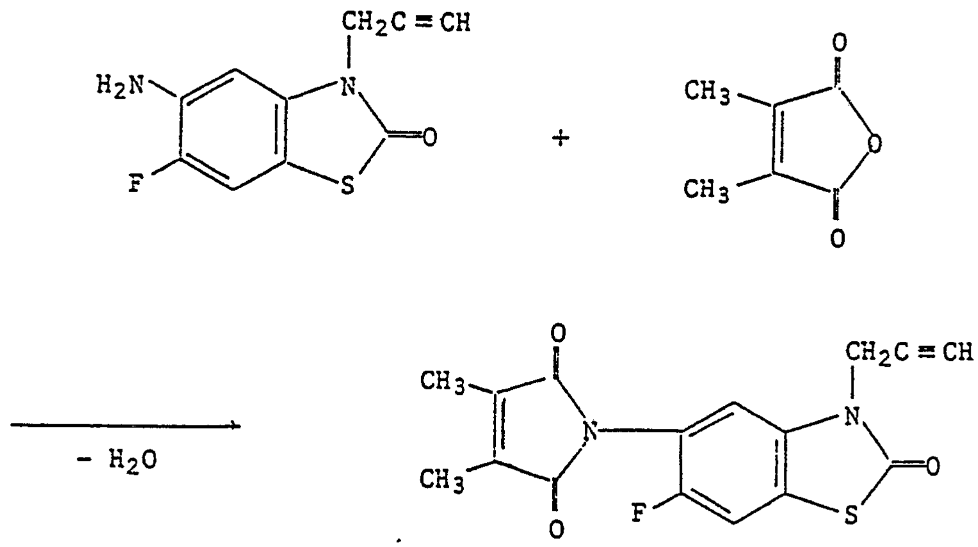


Wenn der in dem Verfahren (c) eingesetzte Ausgangsstoff beispielsweise 5-(3,4-Dimethylmaleinimido)-6-fluoro-3-methylthiomethyl-2-benzothiazolon ist, kann die Oxidations-Reaktion durch das folgende Schema dargestellt werden:

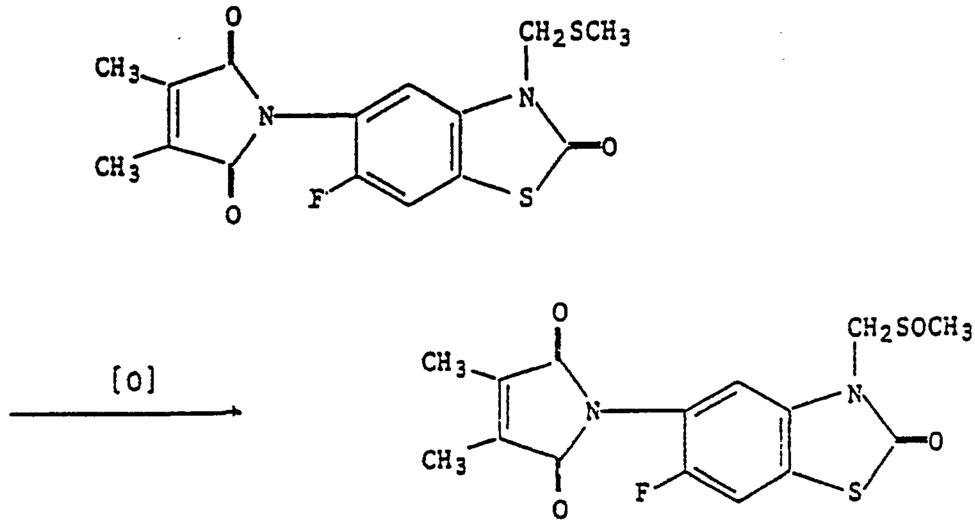


Wenn die in dem Verfahren (d) eingesetzten Ausgangsstoffe beispielsweise 5-(3,4-Dimethylmaleinimido)-6-fluoro-3-(2-oxopropyl)-2-benzothiazolon und Hydroxylamin sind, kann die Reaktion durch das folgende Schema dargestellt werden:

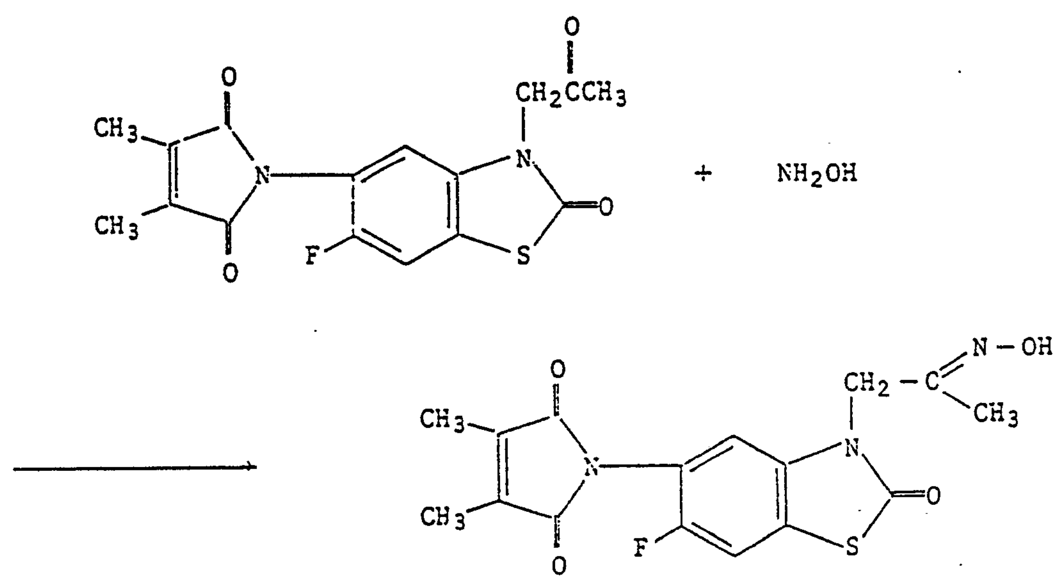


Wenn die in dem Verfahren (e) eingesetzten Ausgangsstoffe beispielsweise 5-(3,4-Dimethylmaleinimido)-6-fluoro-3-(2-hydroxyiminopropyl)-2-benzothiazolon und Iodomethan sind, kann die Reaktion durch das folgende Schema dargestellt werden:

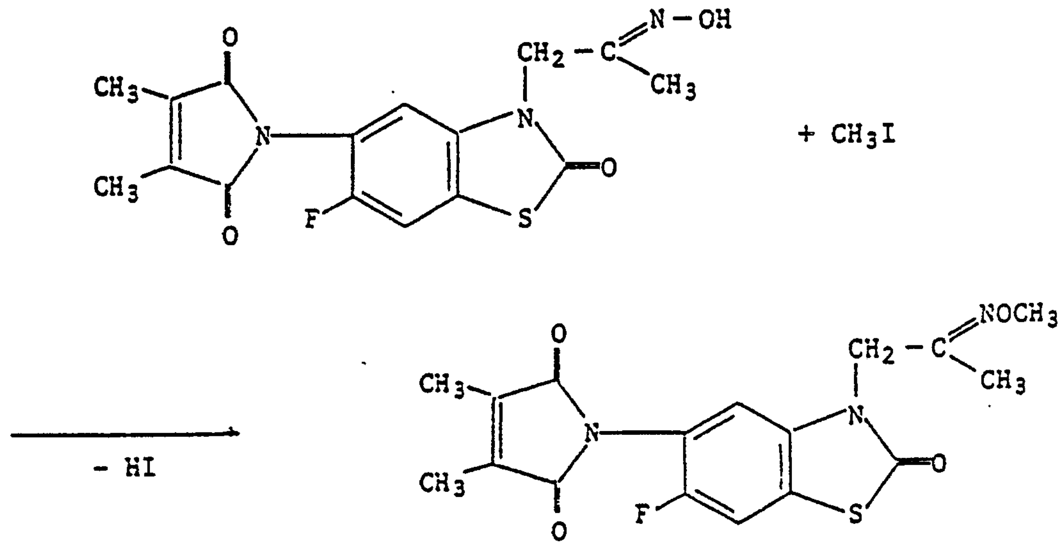


In der Formel (Ia) der in dem Verfahren (a) eingesetzten Ausgangsstoffe hat das Symbol X die im Vorstehenden angegebenen Bedeutungen.

Die Verbindungen der Formel (Ia) fallen in den Bereich der vorliegenden Verbindungen der Formel (I). Die Verbindungen der Formel (Ia) können allgemein mit Hilfe des Verfahrens (b) durch Umsetzung von Verbindungen der Formel (IVa)

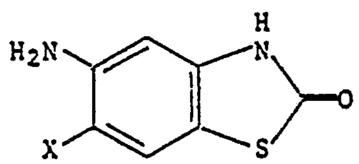

(IVa)

,

in der

X die im Vorstehenden angegebenen Bedeutungen hat, mit 2,3-Dimethylmaleinsäureanhydrid in einer organischen Säure oder in einem Lösungsmittel, das aus Kohlenwasserstoffen mit hohem Siedepunkt besteht, in Gegenwart eines Katalysators wie p-Toluonsulfonsäure hergestellt werden.

Die Verbindungen der Formel (IVa) fallen in den Bereich der Verbindungen der Formel (IV) und sind in der JP-OS 155 903/1987 beschrieben. Die Verbindungen der Formel (IVa) können durch Reduktion einer Verbindung der Formel (VIII)

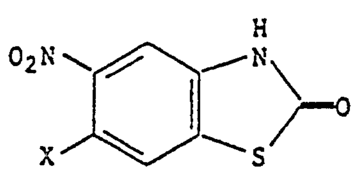

(VIII)

,

in der

X die im Vorstehenden angegebenen Bedeutungen hat, hergestellt werden.

Die Verbindungen der Formel (VIII) sind bekannte Verbindungen.

In der Formel (III) der in dem Verfahren (a) eingesetzten Ausgangsstoffe haben die Symbole $R^1$ und $M^1$ die im Vorstehenden angegebenen Bedeutungen. Vorzugsweise hat $R^1$ die im Vorstehenden als bevorzugt genannten Bedeutungen, und $M^1$ bezeichnet Chlor, Brom oder Iod.

Die Verbindungen der Formel (III) sind bekannte Verbindungen. Beispiele für diese Verbindungen sind

Propargylbromid,

3-Bromopropan,

3-Chloropropen,
Chloromethylmethylether,
Chloromethylmethylsulfid,
Ethylchloroacetat,
Cyclopentylbromoacetat,
Chloroacetonitril
1-Chloro-2-propanon und
Trimethylsilylmethylchlorid.

In der Formel (IV) der in dem Verfahren (b) eingesetzten Ausgangsstoffe haben die Symbole X und $R^1$ die im Vorstehenden angegebenen Bedeutungen, und vorzugsweise haben sie die im Vorstehenden als bevorzugt genannten Bedeutungen.

Die Verbindungen der Formel (IV) können allgemein durch Reduktion von Verbindungen der Formel

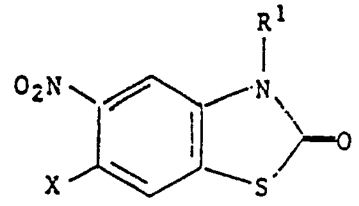

(IX)

hergestellt werden, in der X und $R^1$ die im Vorstehenden angegebenen Bedeutungen haben.

Die Verbindungen der Formel (IX) können durch Umsetzung einer Verbindung der Formel (VIII) mit einer Verbindung der Formel (III) hergestellt werden.

Die in dem Verfahren (c) eingesetzten Verbindungen der Formel (Ib) und die in dem Verfahren (d) eingesetzten Verbindungen der Formel (Ic) fallen in den Bereich der vorliegenden Verbindungen der Formel (I).

In der Formel (VII) der in dem Verfahren (e) eingesetzten Ausgangsstoffe haben die Symbole $R^4$ und $M^1$ die im Vorstehenden angegebenen Bedeutungen, und vorzugsweise haben sie die im Vorstehenden als bevorzugt genannten Bedeutungen.

Die Verbindungen der Formel (VII) sind bekannte Verbindungen. Beispiele für die Verbindung (VII) sind Iodomethan, Iodoethan, 3-Bromopropen und Propargylbromid.

Als geeignete, bei der Durchführung des Verfahrens (a) zu verwendende Verdünnungsmittel können beliebige Arten von inerten organischen Lösungsmitteln genannt werden.

Beispiele für die Verdünnungsmittel sind Wasser, Nitrile wie Acetonitril, Alkohole wie Ethanol, Säureamide wie Dimethylformamid, Sulfoxide wie Dimethylsulfoxid, Ketone wie Aceton und dergleichen.

Das Verfahren (a) kann in Gegenwart einer Base durchgeführt werden. Beispiele für eine solche Base sind Natriumcarbonat, Natriumhydrid, Kaliumcarbonat, Kaliumhydroxid, Natriumhydroxid, Natriummethoxid, Natriumethoxid, Kalium-tert-butoxid und dergleichen.

Die Reaktionstemperatur des Verfahrens (a) kann innerhalb eines Bereichs von beträchtlicher Breite variiert werden. Im allgemeinen wird die Reaktion bei einer Temperatur von etwa 20 °C bis etwa 150 °C, vorzugsweise bei einer Temperatur von etwa 30 °C bis etwa 100 °C, durchgeführt. Es wird bevorzugt, die Reaktion unter normalem Druck durchzuführen, obwohl auch ein höherer oder niedrigerer Druck angewandt werden kann.

Bei dem Verfahren (a) ist es möglich, etwa 1 bis 1,2 mol einer Verbindung (III) auf 1 mol der Verbindungen der Formel (Ia) einzusetzen. Die beiden Ausgangsverbindungen können miteinander in einem inerten Lösungsmittel in Gegenwart einer Base umgesetzt werden, um die angestrebten Verbindungen der Formel (I) zu erhalten.

Als geeignete, bei der Durchführung des Verfahrens (b) zu verwendende Verdünnungsmittel können beliebige Arten inerter Lösungsmittel genannt werden; beispielsweise sind Lösungsmittel zu erwähnen, die denjenigen ähneln, die für das Verfahren (a) beispielhaft genannt wurden. Es ist auch möglich, als Lösungsmittel organische Säuren wie Essigsäure, Propionsäure etc. zu verwenden.

Das Verfahren (b) kann bei Temperaturen innerhalb eines Bereichs von beträchtlicher Breite durchgeführt werden. Es kann beispielsweise bei einer Temperatur von etwa 70 °C bis etwa 280 °C, vorzugsweise von etwa 80 °C bis etwa 140 °C, durchgeführt werden.

In dem Verfahren (b) wird die Reaktion vorzugsweise unter normalem Druck durchgeführt, jedoch ist es auch möglich, unter höherem oder niedrigerem Druck zu arbeiten.

Bei dem Verfahren (b) ist es möglich, etwa bis 1,2 mol 2,3-Dimethylmaleinsäureanhydrid auf 1 mol der

Verbindungen der Formel (IV) einzusetzen. Die Reaktion kann in Gegenwert von Essigsäure durchgeführt werden, so daß die angestrebten Verbindungen der Formel (I) hergestellt werden können.

Die Oxidations-Reaktionen in dem Verfahren (c) können beispielsweise unter Einsatz von Hydrogenperoxid oder Peressigsäure in Essigsäure oder unter Verwendung einer organischen Persäure, z.B. m-Chloroperbenzoesäure, in Chloroform durchgeführt werden.

Das Verfahren (c) kann im allgemeinen bei einer Temperatur von 0 °C bis etwa 120 °C, vorzugsweise von etwa 20 °C bis etwa 110 °C, durchgeführt werden.

Bei dem Verfahren (c) können die Verbindungen der Formel (Ib) beispielsweise mit einer 35-proz. wäßrigen Hydrogenperoxid-Lösung in Essigsäure umgesetzt werden, so daß die angestrebten Verbindungen der Formel (Ia) erhalten werden können. Wie in den nachstehenden Beispielen aufgezeigt wird, kann bei Durchführung der Reaktion bei relativ niedriger Temperatur ein Sulfinyl-Produkt erhalten werden. Wenn die Reaktion bei höherer Temperatur durchgeführt wird, kann ein Sulfonyl-Produkt erhalten werden.

Als geeignete, bei der Durchführung des Verfahrens (d) zu verwendende Verdünnungsmittel seien Alkohole wie Methanol und Ethanol genannt.

Die Reaktion bei dem Verfahren (d) kann bei einer Temperatur von etwa 30 °C bis etwa 90 °C durchgeführt werden. Die Reaktion kann mit Vorteil unter normalem Druck durchgeführt werden, obwohl auch ein höherer oder niedrigerer Druck angewandt werden kann.

Bei dem Verfahren (d) ist es möglich, einen geringfügigen Überschuß an Hydroxylamin auf 1 mol der Verbindungen der Formel (Ic) einzusetzen, um die angestrebten Verbindungen der Formel (Id) herzustellen.

Als geeignete, bei der Durchführung der Alkylierungs-Reaktion in dem Verfahren (e) zu verwendende Verdünnungsmittel können Lösungsmittel genannt werden, die denjenigen ähneln, die für das Verfahren (a) beispielhaft genannt wurden. Bei der Acylierungs-Reaktion können beispielsweise Ether, Benzol, Toluol etc. als Lösungsmittel verwendet werden.

Das Verfahren (e) kann in Gegenwart einer Base durchgeführt werden. Bei der Alkylierungs-Reaktion können beispielsweise die Basen zur Anwendung kommen, die denjenigen ähneln, die für das Verfahren (a) beispielhaft genannt wurden. Bei der Acylierungs-Reaktion ist es möglich, beispielsweise ein organisches Amin wie Pyridin, Triethylamin, N,N-Dimethylanilin oder dergleichen zu verwenden.

Bei dem Verfahren (e) kann die Alkylierungsreaktion unter den gleichen Temperatur-Bedingungen wie im Verfahren (a) durchgeführt werden. Die Acylierungs-Reaktion kann im allgemeinen bei einer Temperatur von etwa -20 °C bis etwa 60 °C, vorzugsweise bei einer Temperatur von etwa 10 °C bis etwa 30 °C, durchgeführt werden. Es wird bevorzugt, eine derartige Reaktion unter normalem Druck durchzuführen, wenngleich auch ein höherer oder niedrigerer Druck angewandt werden können.

Bei der Durchführung des Verfahrens (e) ist es möglich, etwa 1 bis 1,2 mol der Verbindungen der Formel (VII) auf 1 mol der Verbindungen der Formel (Id) einzusetzen und die beiden Ausgangsstoffe miteinander in Gegenwart einer Base umzusetzen, so daß die angestrebten Verbindungen der Formel (Ie) erhalten werden können.

Die erfindungsgemäßen aktiven Verbindungen können als Entlaubungsmittel, Abbrandmittel, Mittel zur Zerstörung breitblättriger Pflanzen und insbesondere als Unkrautvernichtungsmittel eingesetzt werden. Unter "Unkräutern" im weitesten Sinne sind alle Pflanzen zu verstehen, die an Stellen wachsen, wo sie nicht erwünscht sind. Ob die erfindungsgemäßen Substanzen als totale oder selektive Herbizide wirken, hängt im wesentlichen von der verwendeten Menge ab.

Die aktiven Verbindungen gemäß der Erfindung können beispielsweise in Verbindung mit den folgenden Pflanzen verwendet werden:

<u>Dikotyledonen-Unkräuter der Gattungen:</u>

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea.

<u>Dikotyledonen-Kulturen der Gattungen:</u>

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita.

<u>Monokotyledonen-Unkrauter der Gattungen:</u>

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Monokotyledonen-Kulturen der Gattungen:

Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obst gärten, Weinbergen und -gärten, Zitrushainen, Nußbaumpflanzungen, Bananenplantagen, Kaffeeplantagen, Teeplantagen, Gummiplantagen, ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe können in übliche Formulierungen wie Lösungen, Emulsionen, benetzbare Pulver, Suspensionen, Pulver, Schäume, Pasten, Granulat, Aerosole, mit dem Wirkstoff imprägnierte natürliche und synthetische Materialien, sehr feine Kapseln in polymeren Substanzen, Beschichtungsmittel zum Aufbringen auf das Saatgut und Formulierungen, die in Verbindung mit Verbrennungseinrichtungen wie Räucherpatronen, Räucherdosen und Räucherschlangen eingesetzt werden, sowie ULV-Kaltvernebelungs- und Warmvernebelungspräparate umgewandelt werden.

Diese Formulierungen können in bekannter Weise hergestellt werden, beispielsweise durch Vermischen der Wirkstoffe mit Streckmitteln, d.h. flüssigen oder verflüssigten gasförmigen oder festen Verdünnungsmitteln oder Trägern, gegebenenfalls unter Verwendung von grenzflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Unter verflüssigten gasförmigen Verdünnungsmitteln oder Trägern sind Flüssigkeiten zu verstehen, die bei normaler Temperatur und unter normalem Druck gasförmig sein würden, beispielsweise Aerosol-Treibmittel wie halogenierte Kohlenwasserstoffe sowie Butan, Propan, Stickstoff und Kohlenstoffdioxid.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate, Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B. Gummi arabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe oder Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,1 bis 95 Gewichts-%, vorzugsweise von 0,5 bis 90 Gewichts-% des Wirkstoffs.

Die Wirkstoffe gemäß der vorliegenden Erfindung können als solche oder in Form ihrer Formulierungen

auch zur Unkrautbekämpfung als Mischungen mit anderen Herbiziden eingesetzt werden, wobei Fertigformulierungen oder Tankmischungen möglich sind.

Mischungen mit anderen aktiven Verbindungen wie Herbiziden, Fungiziden, Insektiziden, Akariziden, Nematiziden, vogelabweisenden Mitteln, Pflanzennährstoffen sowie Mitteln zur Verbesserung der Bodenstruktur sind ebenfalls möglich.

Die Wirkstoffe können als solche, in Form ihrer Formulierungen oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben oder Streuen.

Die erfindungsgemäßen Wirkstoffe können vor oder nach dem Auflaufen der Pflanzen zur Anwendung gelangen.

Sie können auch vor der Einsaat in den Boden eingearbeitet werden. Insbesondere werden sie nach dem Auflaufen der Pflanzen angewandt.

Die eingesetzten Mengen des Wirkstoffs können innerhalb eines Bereichs von erheblicher Breite variiert werden. Sie hängen wesentlich von der Art der angestrebten Wirkung ab. Im allgemeinen liegen die aufgewandten Mengen des Wirkstoffs zwischen 0,0001 und 3 kg/ha, vorzugsweise zwischen 0,001 und 2 kg/ha.

Die Herstellung und Verwendung der erfindungsgemäßen Verbindungen sind den folgenden Beispielen zu entnehmen.

Herstellungsbeispiele

Beispiel 1

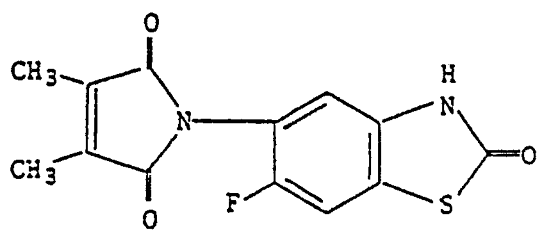

Eine Mischung aus 2,3-Dimethylmaleinsäureanhydrid (13,9 g), 5-Amino-6-fluoro-2-benzothiazolon (18,4 g) und Dowtherm A {ein eutektisches Gemisch aus Diphenylether/Biphenyl (73,5/26,5)}(200 g) wurde 10 min auf eine Temperatur von 200 °C bis 240 °C erhitzt. Die Mischung wurde dann mit p-Toluonsulfonsäure (0,2 g) versetzt und 15 min unter Rückfluß erhitzt. Das Wasser, das sich während dieser Reaktion entwickelte, wurde abdestilliert. Die Reaktionsmischung wurde auf eine Temperatur von 40 °C bis 60 °C abgekühlt, mit n-Hexan (300 ml) versetzt und gerührt. Der dabei gebildete feste Niederschlag wurde durch Filtration abgetrennt, mit n-Hexan (2 x 150 ml) gewaschen und dann in Tetrahydrofuran (1 Liter) gelöst. Die resultierende Lösung wurde filtriert, mit Aktivkohle behandelt und dann unter vermindertem Druck zur Trockne eingedampft. Der auf diese Weise erhaltene feste Stoff wurde mit Ethanol (150 ml) vermischt, 5 min unter Rückfluß erhitzt, danach 1 h auf eine Temperatur von 10 °C bis 15 °C abgekühlt und dann filtriert. Eine auf diese Weise erhaltene kristalline Substanz wurde mit einer kleinen Menge Ethanol gewaschen und getrocknet, so daß das angestrebte 5-(3,4-Dimethylmaleinimido)-6-fluoro-2-benzothiazolon (21 g) erhalten wurde; Schmp. 303-305 °C.

Beispiel 2

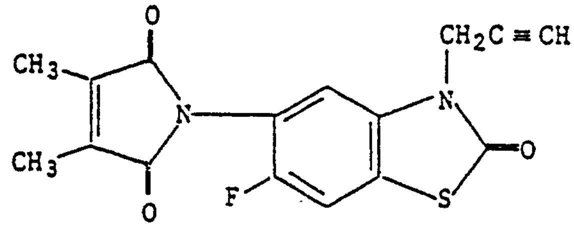

Eine Mischung aus 5-Amino-6-fluoro-3-propargyl-2-benzothiazolon (2,22 g), 2,3-Dimethylmaleinsäureanhydrid (1,3 g) und Essigsäure (50 ml) wurde 1 h bei einer Temperatur von 20 °C bis 30 °C gerührt und dann 1 h unter Rückfluß erhitzt. Die Essigsäure wurde aus der Reaktionsmischung unter vermindertem Druck abdestilliert, und der resultierende Rückstand wurde in Ethylacetat (100 ml) gelöst, mit 5-proz. wäßrigem Kaliumhydroxid, Wasser und gesättigtem wäßrigen Natriumchlorid, in dieser Reihenfolge, gewaschen, über wasserfreiem Natriumsulfat getrocknet und dann unter vermindertem Druck zur Trockne eingedampft.

Das Reaktionsprodukt wurde mittels Silicagel-Säulenchromatographie unter Verwendung eines Elutionsmittels aus Toluol/Tetrahydrofuran (4/1) gereinigt, wonach das angestrebte 5-(3,4-Dimethylmaleinimido)-6-fluoro-3-propargyl-2-benzothiazolon (1,3 g) mit einem Schmp. im Bereich von 227-229 °C erhalten wurde.

Beispiel 3

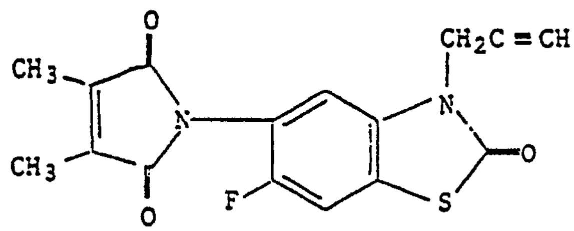

Eine Mischung aus 5-(3,4-Dimethylmaleinimido)-6-fluoro-2-benzothiazolon (2,92 g), Kaliumcarbonat (1,52 g) und Acetonitril (80 ml) wurde 30 min bei einer Temperatur von 45 °C bis 50 °C gerührt. Danach wurde zu der Mischung tropfenweise Propargylbromid (1,3 g) hinzugefügt. Die Reaktionsmischung wurde 3 h bei einer Temperatur von 70 °C bis 80 °C gerührt und dann auf Raumtem peratur abgekühlt. Das Lösungsmittel wurde unter vermindertem Druck abdestilliert, und der Rückstand wurde wie in Beispiel 2 gereinigt, so daß das angestrebte 5-(3,4-Dimethylmaleinimido)-6-fluoro-3-propargyl-2-benzothiazolon (2,8 g) erhalten wurde; Schmp. 227-229 °C.

Beispiel 4

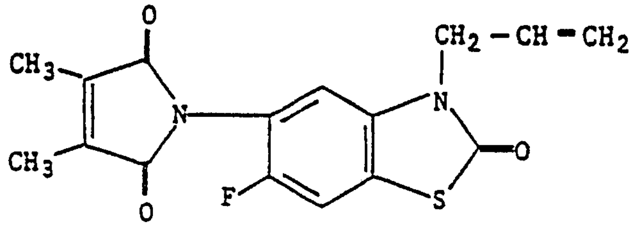

Eine Mischung aus 5-(3,4-Dimethylmaleinimido)-6-fluoro-2-benzothiazolon (2,92 g), Kaliumcarbonat (1,52 g) und Acetonitril (80 ml) wurde 30 min bei einer Temperatur von 50 °C bis 60 °C gerührt. Zu der Mischung wurde tropfenweise 3-Bromopropen (1,34 g) hinzugefügt.

Die Reaktionsmischung wurde 1 h bei einer Temperatur von 50 °C bis 60 °C und dann 3 h unter Rückfluß gerührt. Danach wurde die Reaktionsmischung auf eine Temperatur von 10 °C bis 20 °C abgekühlt und filtriert. Das Filtrat wurde unter vermindertem Druck eingedampft, und der resultierende Rückstand wurde mit Wasser (30 ml) und Ethylacetat (100 ml) vermischt. Die so erhaltene Mischung wurde gerührt. Danach wurde die dabei gebildete organische Schicht abgetrennt. Die abgetrennte organische Schicht wurde mit 5-proz. wäßrigem Kaliumhydroxid und mit gesättigtem wäßrigen Natriumchlorid, in dieser Reihenfolge, gewaschen. Die organische Schicht wurde über wasserfreiem Natriumsulfat getrocknet, und das Ethylacetat wurde unter vermindertem Druck zur Trockne abdestilliert. Der erhaltene Rückstand wurde in einer minimalen Menge von heißem Ethanol gelöst, und die dabei gebildete Lösung wurde über Nacht auf 5 °C bis 10 °C abgekühlt. Danach wurde die so entstandene kristalline Substanz durch Filtration abgetrennt und getrocknet, wodurch das angestrebte 5-(3,4-Dimethylmaleinimido)-6-fluoro-3-(propen-3-yl)-2-benzothiazolon (2,26 g) erhalten wurde; Schmp. 159-162 °C.

Beispiel 5

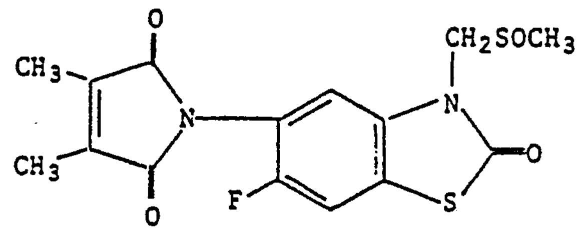

5-(3,4-Dimethylmaleinimido)-6-fluoro-3-methylthiomethyl-2-benzothiazolon (2 g) wurde in Essigsäure (100 ml) gelöst. Zu dieser Lösung wurde tropfenweise 35-proz. wäßriges Hydrogenperoxid (0,65 g) bei einer Temperatur von 10 °C bis 20 °C hinzugefügt. Die Reaktionsmischung wurde 1 h bei einer Temperatur von 10 °C bis 20 °C und dann 8 h bei einer Temperatur von 20 °C bis 30 °C gerührt. Die Mischung wurde mit wäßrigem Eisen(II)-chlorid versetzt, um das überschüssige Hydrogenperoxid zu zersetzen. Danach wurde die Essigsäure abdestilliert. Der so erhaltene Rückstand wurde in Ethylacetat (80 ml) gelöst. Die resultierende Lösung wurde mit gesättigtem wäßrigen Natriumhydrogencarbonat und dann mit gesättigtem wäßrigen Natriumchlorid gewaschen und über wasserfreiem Natriumsulfat getrocknet. Danach wurde Aktivkohle zu der Mischung hinzugefügt, die dann filtriert wurde. Das Filtrat wurde unter vermindertem Druck zur Trockne eingedampft. Der resultierende Rückstand wurde aus Ethanol umkristallisiert, so daß das angestrebte 5-(3,4-Dimethylmaleinimido)-6-fluoro-3-methylsulfinylmethyl-2-benzothiazolon (1 g) erhalten wurde; Sohmp. 250-251,5 °C.

Beispiel 6

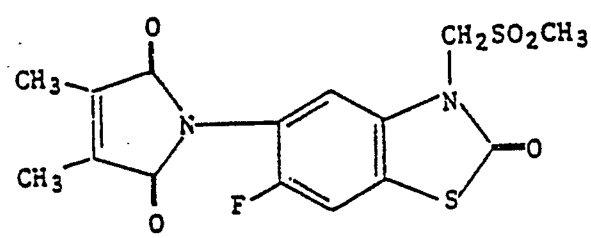

5-(3,4-Dimethylmaleinimido)-6-fluoro-3-methylthiomethyl-2-benzothiazolon (0,704 g) wurde in Essigsäure (50 ml) gelöst. Zu dieser Lösung wurde 35-proz. wäßriges Hydrogenperoxid (0,43 g) bei einer Temperatur von 20 °C bis 30 °C hinzugefügt. Die Reaktionsmischung wurde 1 h bei einer Temperatur von 30 °C bis 40 °C und dann 5 h unter Rückfluß gerührt. Eine kleine Menge wäßriges Eisen(II)-chlorid wurde zugesetzt, um das überschüssige Hydrogenperoxid zu zersetzen. Die Reaktionsmischung wurde dann unter

vermindertem Druck zur Trockne eingedampft. Der so erhaltene Rückstand wurde mit Ethylacetat (80 ml) und Aktivkohle (0,4 g) vermischt, und die Reaktionsmischung wurde gerührt und dann filtriert. Das Filtrat wurde mit gesättigtem wäßrigen Natriumhydrogencarbonat und dann mit gesättigtem wäßrigen Natriumchlorid gewaschen, über wasserfreiem Natriumsulfat getrocknet und unter vermindertem Druck zur Trockne eingedampft. Der erhaltene Rückstand wurde aus Ethanol umkristallisiert, so daß das angestrebte 5-(3,4-Dimethylmaleinimido)-6-fluoro-3-methylsulfonylmethyl-2-benzothiazolon (0,7 g) erhalten wurde; Schmp. 282-283,5 °C.

Beispiel 7

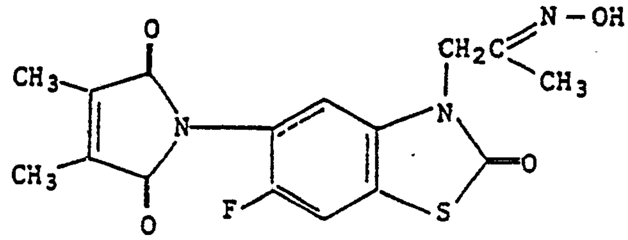

Eine Mischung aus 5-(3,4-Dimethylmaleinimido)-6-fluoro-3-(2-oxopropyl)-2-benzothiazolon (5,5 g), Hydroxylaminhydrochlorid (2 g) und Methanol (220 ml) wurde 2 h unter Rückfluß erhitzt. Das Methanol wurde unter vermindertem Druck abdestilliert, und der Rückstand wurde mit Wasser (50 ml) und Ethylacetat (250 ml) vermischt. Die auf diese Weise gebildete Mischung wurde gerührt, und dann wurde aus ihr eine organische Schicht abgetrennt. Die abgetrennte organische Schicht wurde mit Wasser und mit gesättigtem wäßrigen Natriumchlorid, in dieser Reihenfolge, gewaschen, über wasserfreiem Natriumsulfat getrocknet und unter vermindertem Druck zur Trockne eingedampft. Der erhaltene Rückstand wurde aus Methanol umkristallisiert, so daß das angestrebte 5-(3,4-Dimethylmaleinimido)-6-fluoro-3-(2-hydroxyiminopropyl)-2-benzothiazolon (2,3 g) erhalten wurde; Schmp. 252-253 °C.

Beispiel 8

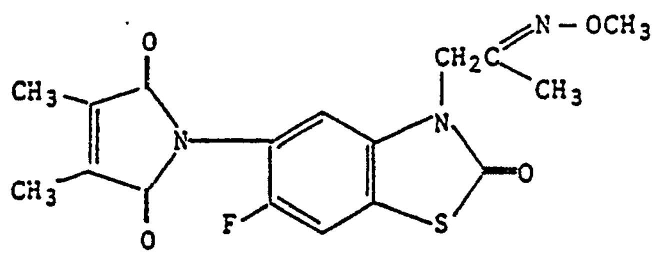

Eine Mischung aus 5-(3,4-Dimethylmaleinimido)-6-fluoro-3-(2-hydroxyiminopropyl)-2-benzothiazolon (0,91 g), Kaliumcarbonat (0,36 g) und Methanol (80 ml) wurde 20 min bei einer Temperatur von 40 °C bis 50 °C gerührt, und Iodomethan (0,43 g) wurde tropfenweise zu dieser Mischung hinzugefügt. Die Reaktionsmischung wurde 3 h unter Rückfluß gerührt und einem Arbeitsgang der Destillation zur Entfernung der niedrigsiedenden Substanzen aus ihr unterworfen. Der Rückstand wurde mit Wasser (10 ml) und Ethylacetat (60 ml) vermischt, und die erhaltene Mischung wurde gerührt. Eine dabei gebildete organische Schicht wurde abgetrennt. Die abgetrennte organische Schicht wurde mit Wasser und mit gesättigtem wäßrigen Natriumchlorid, in dieser Reihenfolge, gewaschen, über wasserfreiem Natriumsulfat getrocknet und dann unter vermindertem Druck zur Trockne eingedampft. Der erhaltene Rückstand wurde mittels Silicagel-Säulenchromatographie unter Verwendung eines Toluol/Tetrahydrofuran-Gemischs (5/1) als Elutionsmittel gereinigt, wonach das angestrebte 5-(3,4-Dimethylmaleinimido)-6-fluoro-3-(2-methoxyiminopropyl)-2-benzothiazolon (0,27 g) erhalten wurde; Schmp. 282-283,5 °C.

Beispiel 9

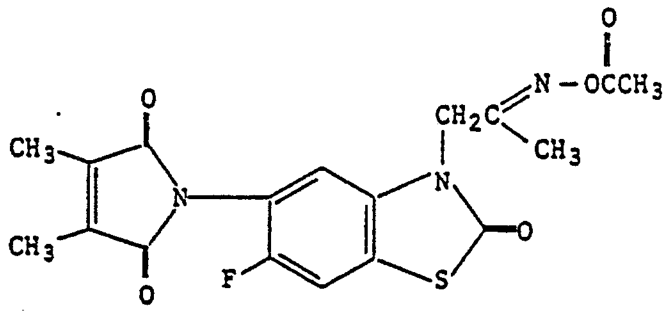

5-(3,4-Dimethylmaleinimido)-6-fluoro-3-(2-hydroxyiminopropyl)-2-benzothiazolon (0,91 g) wurde in Tetrahydrofuran (30 ml) gelöst, und die dabei gebildete Mischung wurde mit Triethylamin (0,26 g) vermischt und auf eine Temperatur von -10 °C bis 0 °C gekühlt. Zu der Mischung wurde tropfenweise eine Lösung von Acetylchlorid (0,2 g) in Tetrahydrofuran (10 ml) bei einer Temperatur von -10 °C bis 0 °C hinzugefügt. Dann wurde die Reaktionsmischung 2 h bei einer Temperatur von 0 °C bis 10 °C gerührt und danach über Nacht bei einer Temperatur von 20 °C bis 30 °C stehen gelassen. Das Tetrahydrofuran wurde unter vermindertem Druck abdestilliert, und der Rückstand wurde mit Wasser (10 ml) und Ethylacetat (30 ml) vermischt. Die resultierende Mischung wurde gerührt, und eine dabei gebildete organische Schicht wurde abgetrennt. Die abgetrennte organische Schicht wurde mit gesättigtem wäßrigen Natriumhydrogencarbonat und mit gesättigtem wäßrigen Natriumchlorid, in dieser Reihenfolge, gewaschen, über wasserfreiem Natriumsulfat getrocknet. Das Lösungsmittel wurde unter vermindertem Druck abdestilliert, so daß das angestrebte 3-(2-Acetoxyiminopropyl)-5-(3,4-dimethylmaleinimido)-6-fluoro-2-benzothiazolon (0,8 g) als viskose Flüssigkeit erhalten wurde.

In der gleichen Weise, wie sie in den Beispielen 1 bis 9 beschrieben ist, wurden verschiedenartige erfindungsgemäße Verbindungen der Formel (I) hergestellt. Diese Verbindungen sind, zusammen mit den Verbindungen der Beispiele 1 bis 9, in der Tabelle 1 aufgeführt.

## Tabelle 1

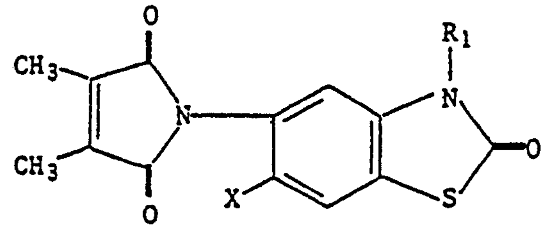

| Verbindung Nr. | X | $R^1$ | Schmp. (°C) |
|---|---|---|---|
| 1 | H | H | 300 - 305 |
| 2 | H | $-C_3H_7$ | |
| 3 | H | $-CH_2CH=CH_2$ | |
| 4 | H | Cl<br>$-CH_2C=CH_2$ | |
| 5 | H | $-CH_2C\equiv CH$ | 266 - 267 |
| 6 | H | O<br>$-CH_2C-CH_3$ | |
| 7 | F | H | 303 - 305 |
| 8 | F | $-CH_3$ | 255 - 260 |
| 9 | F | $-C_2H_5$ | 207 - 210 |
| 10 | F | $-C_3H_7$-iso | 163 - 166 |
| 11 | F | $-C_3H_7$ | 172 - 173 |
| 12 | F | $-C_4H_9$-n | |
| 13 | F | $-C_4H_9$-sec | |

Tabelle 1 - Fortsetzung

| Verbindung Nr. | X | $R^1$ | Schmp. (°C) |
|---|---|---|---|
| 14 | F | $-CH_2CH_2Cl$ | |
| 15 | F | $-CH_2CH_2CH_2Cl$ | |
| 16 | F | $-CH_2CH=CH_2$ | 159 - 162 |
| 17 | F | 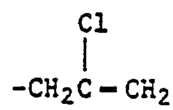 | 189 - 192 |
| 18 | F | $-CH_2CH=CHCl$ | |
| 19 | F | 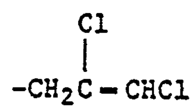 | |
| 20 | F | 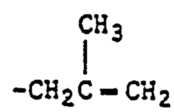 | 177 - 179 |
| 21 | F | $-CH_2C\equiv CH$ | 227 - 229 |
| 22 | F | $-CH_2C\equiv CCH_3$ | |
| 23 | F | $-CH(CH_3)C\equiv CH$ | 191 - 199 |
| 24 | F | $-CH_2C\equiv N$ | 214 - 218 |
| 25 | F | 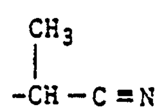 | 188 - 191 |
| 26 | F | 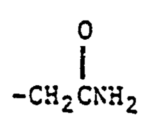 | |
| 27 | F | 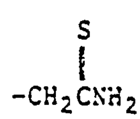 | |

Tabelle 1 - Fortsetzung

| Verbindung Nr. | X | $R^1$ | Schmp. (°C) |
|---|---|---|---|
| 28 | F | $-CH_2OCH_3$ | 202 - 207 |
| 29 | F | $-CH_2OC_2H_5$ | |
| 30 | F | $-CH_2OC_3H_7$ | |
| 31 | F | $-CH_2SCH_3$ | 207 - 209 |
| 32 | F | 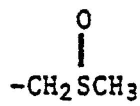 | 250 - 251.5 |
| 33 | F | 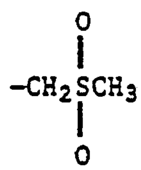 | 282 - 283.5 |
| 34 | F | $-CH_2SC_2H_5$ | |
| 35 | F | 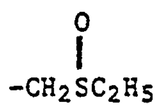 | |
| 36 | F | $-CH_2S(O)_2C_2H_5$ | |
| 37 | F | $-CH_2CH_2SC_2H_5$ | |
| 38 | F | $-CH_2CH_2S(O)C_2H_5$ | |
| 39 | F | $-CH_2CH_2S(O)_2C_2H_5$ | |
| 40 | F | 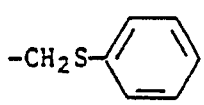 | |
| 41 | F | 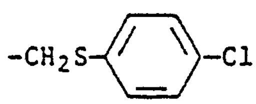 | |

Tabelle 1 - Fortsetzung

| Verbindung Nr. | X | $R^1$ | Schmp. (°C) |
|---|---|---|---|
| 42 | F | 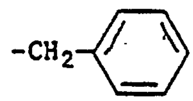 | 236 - 237 |
| 43 | F | 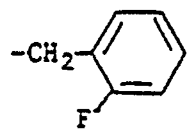 | |
| 44 | F | 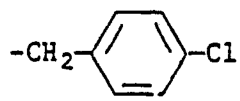 | |
| 45 | F | 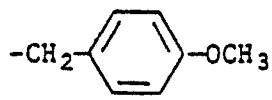 | |
| 46 | F | 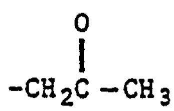 | 252 - 253 |
| 47 | F | 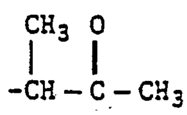 | |
| 48 | F | 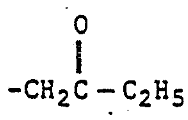 | |
| 49 | F | 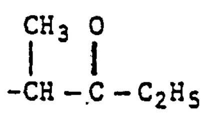 | |
| 50 | F | 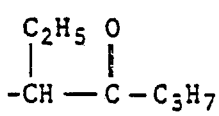 | |
| 51 | F | 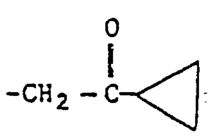 | |

Tabelle 1 - Fortsetzung

| Verbindung Nr. | X | $R^1$ | Schmp. (°C) |
|---|---|---|---|
| 52 | F | 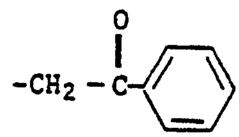 | |
| 53 | F | 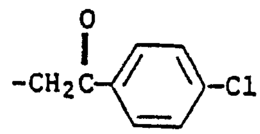 | |
| 54 | F | 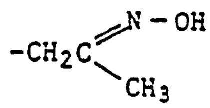 | 252 - 253 |
| 55 | F | 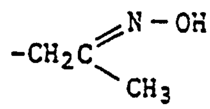 | 194 - 196 |
| 56 | F | 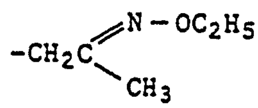 | |
| 57 | F | 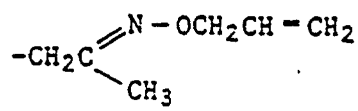 | |
| 58 | F | 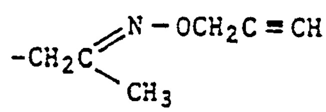 | |
| 59 | F | 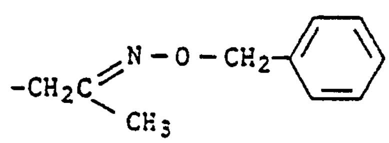 | |
| 60 | F | 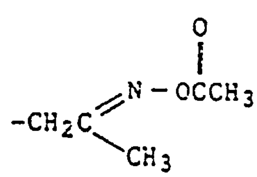 | |

Tabelle 1 - Fortsetzung

| Verbindung Nr. | X | $R^1$ | Schmp. (°C) |
|---|---|---|---|
| 61 | F | 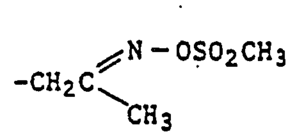 | |
| 62 | F | $-CH_2Si(CH_3)_3$ | |
| 63 | F | $-CH_2COOC_2H_5$ | 154 - 157 |
| 64 | F | 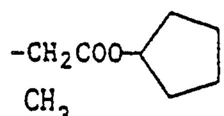 | |
| 65 | F | $CH_3$<br>$-CHCOOC_2H_5$ | 131 - 132 |

Biologische Test-Beispiele

Zum Vergleich eingesetzte, bekannte Verbindung (E-1):

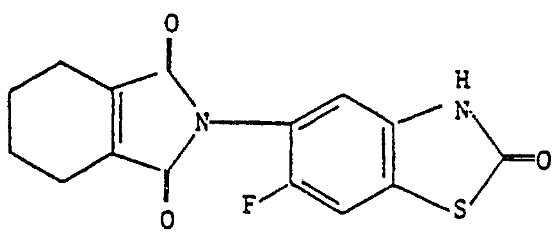

(Die Vergleichs-Verbindung ist in der JP-OS 155 276/1987 beschrieben).

Beispiel 10

Test gegen Unkräuter auf höher gelegenem Ackerboden durch Bodenbehandlung vor dem Auflaufen:

Herstellung eines Wirkstoff-Präparats

Träger: 5 Gewichtsteile Aceton;
Emulgator: 1 Gewichtsteil Benzyloxypolyglycolether.

Ein Wirkstoff-Präparat in Form eines emulgierbaren Konzentrats wurde erhalten durch Vermischen von 1 Gewichtsteil der aktiven Verbindung mit den oben angegebenen Mengen des Trägers und des emulgierenden Mittels. Eine vorher festgelegte Menge des Präparats wurde mit Wasser verdünnt.

Test-Verfahren

In einem Gewächshaus wurden Sojabohnen-Samen in Töpfe von 500 $cm^2$ Fläche, die mit Ackererde gefüllt waren, eingesät, und Erde, die Samen von Hühnerhirse (Echinocloa crus-galli), grauem Amaranth (Amaranthus lividus L.) und Gänsefuß (Chenopodium album L.) enthielt, wurde über die Erde in den Töpfen in einer Tiefe von 1 cm gestreut.

Einen Tag nach der Einsaat wurde eine Test-Chemikalie in einer vorher festgelegten Konzentration, hergestellt wie im Vorstehenden beschrieben, gleichmäßig über die Oberflächenschicht des Bodens in jedem der Test-Töpfe gesprüht.

Vier Wochen nach dem Sprühen wurden die herbizide Wirkung und die Phytotoxizität gegenüber den Nutzpflanzen anhand der folgenden Skala von 0 bis 5 bewertet:

| Bewertung | Herbizide Wirkung (bewertet als Unkrautvernichtungs-Verhältnis, bezogen auf eine unbehandelte Fläche) |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |
| **Bewertung** | **Phytotoxizität gegenüber Nutzpflanzen (bewertet unter Bezug auf die unbehandelte Fläche)** |
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Ergebnisse sind in Tabelle 2 aufgeführt.

Tabelle 2

| Aktive Verbindung Nr. | Wirkstoff-Menge kg/ha | **Herbizide Wirkung** gegen Unkräuter | | | Phytotoxizität gegen Sojabohnenpflanzen |
|---|---|---|---|---|---|
| | | Hühnerhirse | Grauer Amaranth | Gänsefuß | |
| 17 | 0,25 | 4 | 5 | 5 | 0 |
| | 0,125 | 3 | 5 | 5 | 0 |
| 21 | 0,25 | 5 | 5 | 5 | 0 |
| | 0,125 | 4 | 5 | 5 | 0 |
| 46 | 0,25 | 4 | 5 | 5 | 1 |
| | 0,125 | 3 | 5 | 5 | 0 |
| Kontroll-Verbindung | | | | | |
| E-1 | 0,25 | 0 | 4 | 3 | 0 |
| | 0,125 | 0 | 3 | 1 | 0 |

Beispiel 11

Test gegen Unkräuter auf höher gelegenem Ackerboden durch Laub-Behandlung:

In einem Gewächshaus wurden Mais-Samen in Töpfe von 500 $cm^2$ Fläche, die mit Ackererde gefüllt waren, eingesät, und Erde, die Samen von Polygonum (Polygonum blumei Meisn.), grauem Amaranth (Amaranthus lividus L.) und Gänsefuß (Chenopodium album L.) enthielt, wurde über die Erde in den Töpfen in einer Tiefe von 1 cm gestreut.

Nach der Einsaat wurden die Pflanzen 14 Tage angezogen, und eine Test-Chemikalie in einer vorher festgelegten Konzentration, hergestellt wie in Beispiel 10, wurde gleichmäßig über Test-Pflanzen in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 10 geprüft. Die Ergebnisse sind in Tabelle 3 aufgeführt.

Tabelle 3

| Aktive Verbindung Nr. | Wirkstoff-Menge kg/ha | **Herbizide Wirkung** gegen Unkräuter | | | Phytotoxizität gegen Maispflanzen |
|---|---|---|---|---|---|
| | | Polygonum | Grauer Amaranth | Gänsefuß | |
| 17 | 0,125 | 5 | 5 | 5 | 1 |
| | 0,06 | 4 | 5 | 5 | 0 |
| 21 | 0,125 | 5 | 5 | 5 | 1 |
| | 0,06 | 4 | 5 | 5 | 0 |
| 32 | 0,125 | 5 | 5 | 5 | 1 |
| | 0,06 | 5 | 5 | 5 | 0 |
| Kontroll-Verbindung | | | | | |
| E-1 | 0,125 | 3 | 3 | 2 | 1 |
| | 0,06 | 1 | 2 | 1 | 0 |

**Ansprüche**

1. Benzothiazolone der Formel (I)

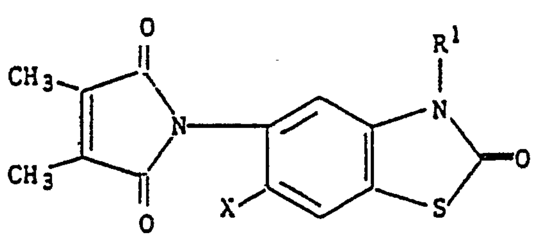

(I)

in der

X Wasserstoff oder Halogen darstellt,

$R^1$ Wasserstoff, Alkyl mit 1 bis 5 Kohlenstoff-Atomen, das gegebenenfalls mit Halogen substituiert ist, Alkenyl mit 3 bis 5 Kohlenstoff-Atomen, das gegebenenfalls mit Halogen substituiert ist, Alkinyl mit 3 bis 4 Kohlenstoff-Atomen, das gegebenenfalls mit Halogen substituiert ist, Alkoxyalkyl mit insgesamt 2 bis 5 Kohlenstoff-Atomen, Alkylthioalkyl mit insgesamt 2 bis 5 Kohlenstoff-Atomen, Alkylsulfinylalkyl mit insgesamt 2 bis 5 Kohlenstoff-Atomen, Alkylsulfonylalkyl mit insgesamt 2 bis 5 Kohlenstoff-Atomen, Arylthioalkyl

mit insgesamt 7 bis 8 Kohlenstoff-Atomen, vorzugsweise Phenylthioalkyl mit 1 oder 2 Kohlenstoff-Atomen im Alkyl-Teil und gegebenenfalls mit Halogen substituiert, Cyanoalkyl mit einer Alkyl-Struktureinheit mit 1 bis 2 Kohlenstoff-Atomen, Carbamoylmethyl, Thiocarbamoylmethyl, Alkoxycarbonylalkyl mit insgesamt 3 bis 8 Kohlenstoff-Atomen, Cycloalkoxycarbonylmethyl mit einer Cycloalkyl-Struktureinheit mit 3 bis 7 Kohlenstoff-Atomen, Trialkylsilylmethyl mit insgesamt 4 bis 10 Kohlenstoff-Atomen oder einen Rest einer der folgenden Formeln

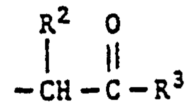 , 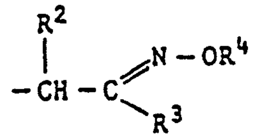 und 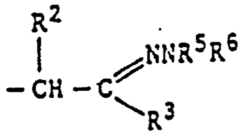

darstellt, in denen

$R^2$ Wasserstoff oder Alkyl mit bis 3 Kohlenstoff-Atomen darstellt,

$R^3$ Alkyl mit 1 bis 4 Kohlenstoff-Atomen, Cycloalkyl mit 3 bis 6 Kohlenstoff-Atomen oder Phenyl, das gegebenenfalls mit Halogen substituiert ist, darstellt,

$R^4$ Wasserstoff, Alkyl mit 1 bis 4 Kohlenstoff-Atomen, Alkenyl mit 3 bis 4 Kohlenstoff-Atomen, Alkinyl mit 3 bis 4 Kohlenstoff-Atomen, Aralkyl mit insgesamt 7 bis 9 Kohlenstoff-Atomen, Alkylcarbonyl mit einer Alkyl-Struktureinheit mit i bis 4 Kohlenstoff-Atomen oder Alkansulfonyl mit 1 bis 4 Kohlenstoff-Atomen darstellt und

$R^5$ und $R^6$ jeweils Wasserstoff oder Alkyl mit 1 bis 4 Kohlenstoff-Atomen darstellen.

2. Verbindungen der Formel (I) nach Anspruch 1, worin

X Wasserstoff oder Fluor darstellt,

$R^1$ Wasserstoff, Alkyl mit 1 bis 4 Kohlenstoff-Atomen, das gegebenenfalls mit Chlor substituiert ist, Alkenyl mit 3 bis 4 Kohlenstoff-Atomen, das gegebenenfalls mit Chlor substituiert ist, Alkinyl mit 3 bis 4 Kohlenstoff-Atomen, das gegebenenfalls mit Chlor substituiert ist, Alkoxyalkyl mit insgesamt 2 bis 4 Kohlenstoff-Atomen, Alkylthioalkyl mit insgesamt 2 bis 3 Kohlenstoff-Atomen, Alkylsulfinylalkyl mit insgesamt 2 bis 4 Kohlenstoff-Atomen, Alkylsulfonylalkyl mit insgesamt 2 bis 4 Kohlenstoff-Atomen, Phenylthiomethyl, das gegebenenfalls im Phenyl-Teil mit Chlor substituiert ist, Cyanomethyl, Carbamoylmethyl, Thiocarbamoylmethyl, Alkoxycarbonylalkyl mit insgesamt 3 bis 6 Kohlenstoff-Atomen, Cycloalkoxycarbonylmethyl mit einer Cycloalkyl-Struktureinheit mit 3 bis 6 Kohlenstoff-Atomen, Trimethylsilylmethyl oder einen Rest einer der folgenden Formeln

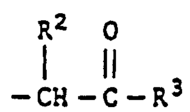 , 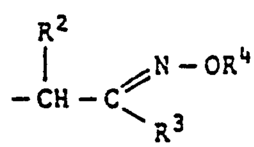 und 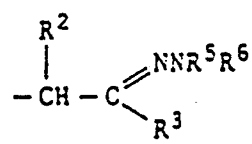

darstellt, in denen

$R^2$ Wasserstoff, Methyl oder Ethyl darstellt,

$R^3$ Alkyl mit 1 bis 3 Kohlenstoff-Atomen, Cycloalkyl mit 3 bis 5 Kohlenstoff-Atomen oder Phenyl, das gegebenenfalls mit Fluor und/oder mit Chlor substituiert ist, darstellt,

$R^4$ Wasserstoff, Alkyl mit 1 bis 3 Kohlenstoff-Atomen, Allyl, Propargyl, Benzyl, Alkylcarbonyl mit einer Alkyl-Struktureinheit mit 1 bis 3 Kohlenstoff-Atomen oder Alkansulfonyl mit 1 bis 2 Kohlenstoff-Atomen darstellt und

$R^5$ und $R^6$ jeweils Wasserstoff oder Alkyl mit 1 bis 2 Kohlenstoff-Atomen darstellen.

3. Verbindungen der Formel (I) nach Anspruch 1, worin

X Fluor darstellt,

$R^1$ Wasserstoff, Alkyl mit 1 bis 3 Kohlenstoff-Atomen, das gegebenenfalls mit Chlor substituiert ist, Allyl, das gegebenenfalls mit Chlor substituiert ist, Propargyl, Alkoxymethyl mit einer Alkoxy-Struktureinheit mit 1 bis 3 Kohlenstoff-Atomen, Alkylthiomethyl mit einer Alkylmercapto-Struktureinheit mit 1 bis 2 Kohlenstoff-Atomen, Alkylsulfinylmethyl mit einer Alkylsulfinyl-Struktureinheit mit 1 bis 2 Kohlenstoff-Atomen, Alkylsulfonylmethyl mit einer Alkylsulfonyl-Struktureinheit mit 1 bis 2 Kohlenstoff-Atomen, Cyanomethyl oder einen Rest der Formel

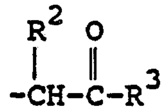

darstellt, in denen

$R^2$ Wasserstoff oder Methyl darstellt und

$R^3$ Methyl oder Ethyl darstellt.

4. Verbindungen der Formel (I) nach den Ansprüchen 1 bis 3, nämlich 5-(3,4-Dimethylmaleinimido)-6-fluoro-3-propargyl-2-benzothiazolon der Formel

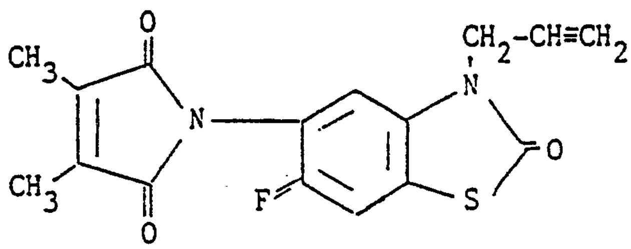

3-Allyl-5 (3,4-dimethylmaleinimido)-6-fluoro-2-benzothiazolon der Formel

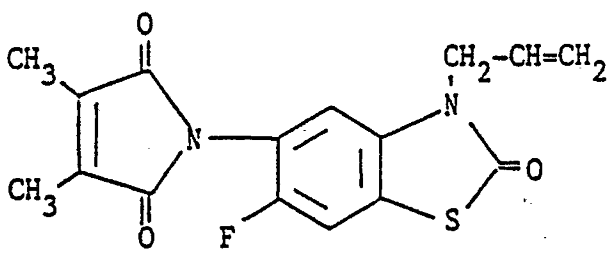

5-(3,4-Dimethylmaleinimido)-6-fluoro-3-propyl-2-benzothiazolon der Formel

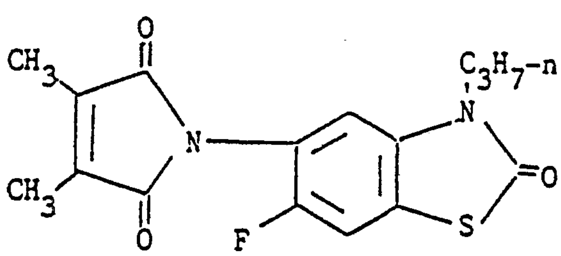

3-Cyanomethyl-5-(3,4-dimethylmaleinimido)-6-fluoro-2-benzothiazolon der Formel

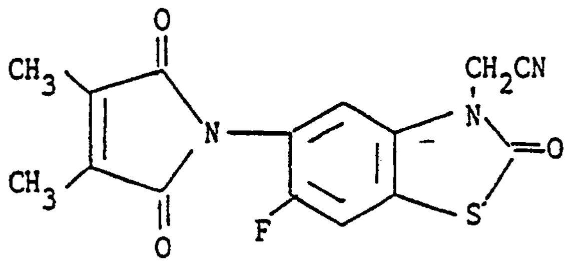

,

5-(3,4-Dimethylmaleinimido)-6-fluoro-3-methylthiomethyl-2-benzothiazolon der Formel

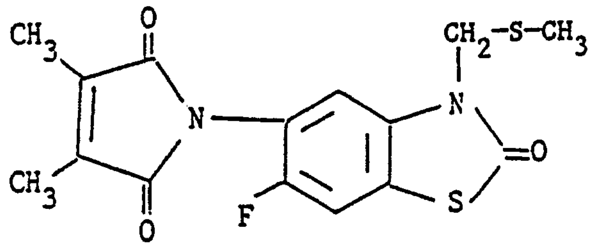

,

5-(3,4-Dimethylmaleinimido)-6-fluoro-3-methylsulfinylmethyl-2-benzothiazolon der Formel

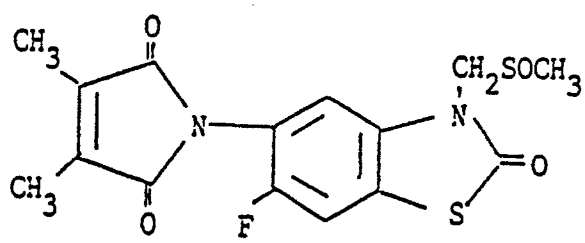

und
5-(3,4-Dimethylmaleinimido)-6-fluoro-3-methylsulfonylmethyl-2-benzothiazolon der Formel

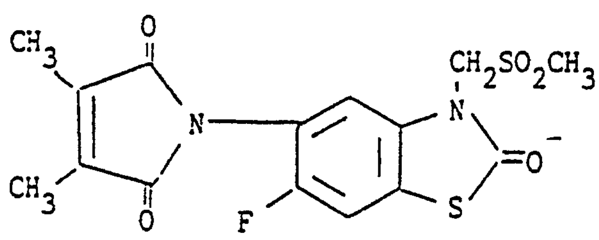

5. Verfahren zur Herstellung von Benzothiazolonen der Formel (I)

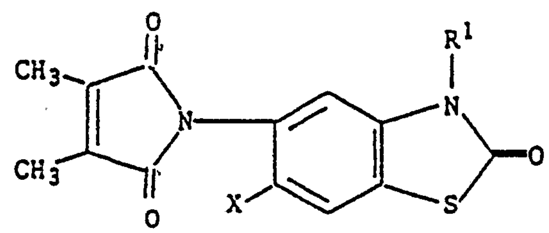

(I)

,

in der
X  Wasserstoff oder Halogen darstellt,
$R^1$  Wasserstoff, Alkyl mit 1 bis 5 Kohlenstoff-Atomen, das gegebenenfalls mit Halogen substituiert ist, Alkenyl mit 3 bis 5 Kohlenstoff-Atomen, das gegebenenfalls mit Halogen substituiert ist, Alkinyl mit 3 bis 4 Kohlenstoff-Atomen, das gegebenenfalls mit Halogen substituiert ist, Alkoxyalkyl mit insgesamt 2 bis 5 Kohlenstoff-Atomen, Alkylthioalkyl mit insgesamt 2 bis 5 Kohlenstoff-Atomen, Alkylsulfinylalkyl mit insgesamt 2 bis 5 Kohlenstoff-Atomen, Alkylsulfonylalkyl mit insgesamt 2 bis 5 Kohlenstoff-Atomen, Arylthioalkyl mit insgesamt 7 bis 8 Kohlenstoff-Atomen, und im Phenylteil gegebenenfalls mit Halogen substituiert, Cyanoalkyl mit einer Alkyl-Struktureinheit mit 1 bis 2 Kohlenstoff-Atomen, Carbamoylmethyl, Thiocarbamoylmethyl, Alkoxycarbonylalkyl mit insgesamt 3 bis 8 Kohlenstoff-Atomen, Cycloalkoxycarbonylmethyl mit einer Cycloalkyl-Struktureinheit mit 3 bis 7 Kohlenstoff-Atomen, Trialkylsilylmethyl mit insgesamt 4 bis 10 Kohlenstoff-Atomen oder einen Rest einer der folgenden Formeln

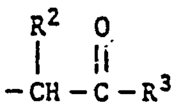 , 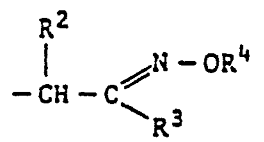 und 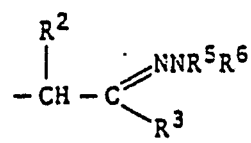

darstellt, in denen
$R^2$  Wasserstoff oder Alkyl mit 1 bis 3 Kohlenstoff-Atomen darstellt,
$R^3$  Alkyl mit 1 bis 4 Kohlenstoff-Atomen, Cycloalkyl mit 3 bis 6 Kohlenstoff-Atomen oder Phenyl, das gegebenenfalls mit Halogen substituiert ist, darstellt,
$R^4$  Wasserstoff, Alkyl mit 1 bis 4 Kohlenstoff-Atomen, Alkenyl mit 3 bis 4 Kohlenstoff-Atomen, Alkinyl mit 3 bis 4 Kohlenstoff-Atomen, Aralkyl mit insgesamt 7 bis 9 Kohlenstoff-Atomen,
Alkylcarbonyl mit einer Alkyl-Struktureinheit mit 1 bis 4 Kohlenstoff-Atomen oder Alkansulfonyl mit 1 bis 4 Kohlenstoff-Atomen darstellt und
$R^5$  und $R^6$ jeweils Wasserstoff oder Alkyl mit 1 bis 4 Kohlenstoff-Atomen darstellen,
dadurch gekennzeichnet, daß
a) Verbindungen Formel (Ia)

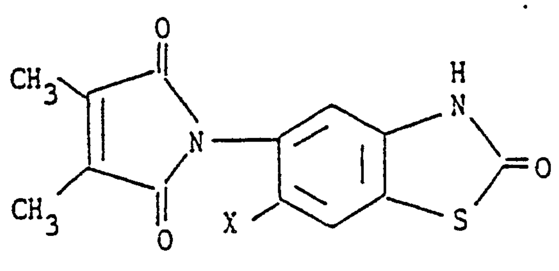

(Ia)

,

in der
X  die im Vorstehenden angegebenen Bedeutungen hat,
mit Verbindungen der Formel (III)
$M^1 - R^1$  (III),
in der
$R^1$  die im Vorstehenden angegebenen Bedeutungen hat und
$M^1$  Halogen ist,
in Gegenwart inerter Lösungsmittel und erforderlichenfalls in Gegenwart von Basen umgesetzt werden, oder

b) Verbindungen der Formel (IV)

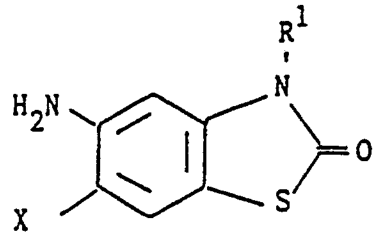

(IV)

,

in der
X und $R^1$ die im Vorstehenden angegebenen Bedeutungen haben,
mit 2,3-Dimethylmaleinsäureanhydrid in Gegenwart inerter Lösungsmittel umgesetzt werden, oder

c) in dem Fall, in dem $R^1$ Alkylsulfinlyalkyl mit insgesamt 2 bis 5 Kohlenstoff-Atomen oder Alkylsulfonlyalkyl mit insgesamt 2 bis 5 Kohlenstoff-Atomen ist, Verbindungen der Formel (Ib)

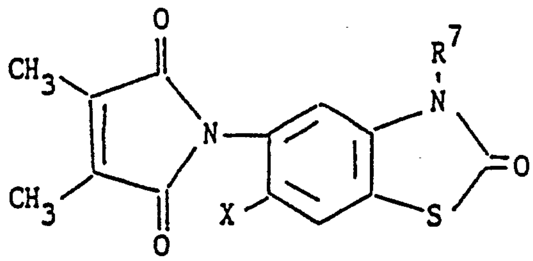

(Ib)

,

in der
X die im Vorstehenden angegebenen Bedeutungen hat und
$R^7$ Alkylthioalkyl mit insgesamt 2 bis 5 Kohlenstoff-Atomen ist,
in Gegenwart inerter Lösungsmittel oxidiert werden, oder

d) in dem Fall, in dem $R^1$

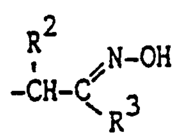

ist,
Verbindungen der Formel (Ic)

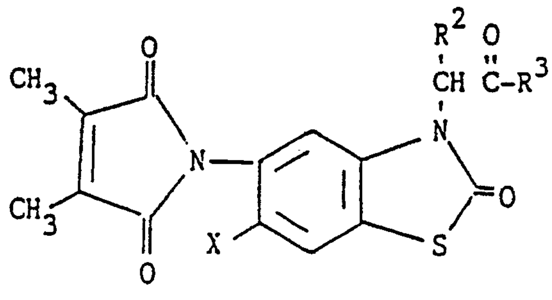

(Ic)

,

in der
X, $R^2$ und $R^3$ die im Vorstehenden angegebenen Bedeutungen haben,
mit Hydroxylamin in Gegenwart inerter Lösungsmittel umgesetzt werden, oder

e) in dem Fall, in dem $R^1$

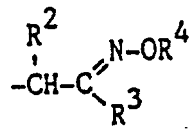

ist,
Verbindungen der Formel (Id)

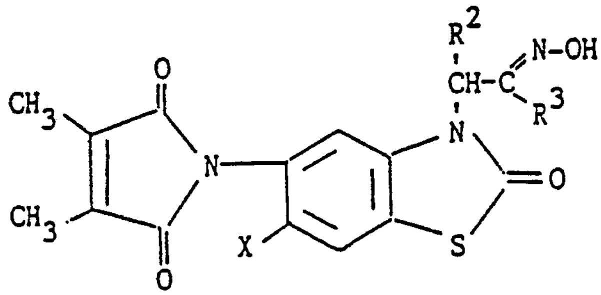

(Id)

,

in der
X, $R^2$ und $R^3$ die im Vorstehenden angegebenen Bedeutungen haben,
mit Verbindungen der Formel (VII)
$M^1 - R^4$ (VII),
in der
$R^4$ und $M^1$ die im Vorstehenden angegebenen Bedeutungen haben,
in Gegenwart inerter Lösungsmittel und erforderlichenfalls in Gegenwart von Basen umgesetzt werden.

6. Herbizide Zusammensetzungen, dadurch gekennzeichnet, daß sie wenigstens ein Benzothiazolon der Formel (I) nach irgendeinem der Ansprüche 1 bis 5 enthalten.

7. Verfahren zur Unkrautbekämpfung, dadurch gekennzeichnet, daß man Benzothiazolone der Formel (I) nach irgendeinem der Ansprüche 1 bis 5 auf Unkräuter und/oder ihren Lebensraum einwirken läßt.

8. Verwendung von Benzothiazolonen der Formel (I) nach irgendeinem der Ansprüche 1 bis 5 zur Unkrautbekämpfung.

9. Verfahren zur Herstellung herbizider Zusammensetzungen, dadurch gekennzeichnet, daß Benzothiazolone der Formel (I) nach irgendeinem der Ansprüche 1 bis 5 mit Streckmitteln und/oder grenzflächenaktiven Mitteln vermischt werden.